(19) Europäisches Patentamt
European Patent Office
Office européen des brevets

(11) Veröffentlichungsnummer: **0 164 654**
**A2**

# (12) EUROPÄISCHE PATENTANMELDUNG

(21) Anmeldenummer: **85106652.2**

(22) Anmeldetag: **30.05.85**

(51) Int. Cl.⁴: **C 07 K 7/06**
C 07 K 5/10, A 61 K 37/02

(30) Priorität: **09.06.84 DE 3421614**

(43) Veröffentlichungstag der Anmeldung:
**18.12.85 Patentblatt 85/51**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **König, Wolfgang, Dr.**
**Eppsteiner Strasse 25**
**D-6238 Hofheim am Taunus(DE)**

(72) Erfinder: **Geiger, Rolf, Prof. Dr.**
**Heinrich-Bleicher-Strasse 33**
**D-6000 Frankfurt am Main 50(DE)**

(72) Erfinder: **Obermeier, Rainer, Dr.**
**Langenhainer Weg 14**
**D-6234 Hattersheim am Main(DE)**

(72) Erfinder: **Müllner, Hubert, Dr.**
**Pestalozzistrasse 4**
**D-6233 Kelkheim (Taunus)(DE)**

## (54) Verfahren zur Herstellung von Pentapeptiden mit Wirkung auf das Immunsystem und Zwischenprodukte dieses Verfahrens.

(57) Die Erfindung betrifft ein Verfahren zur Herstellung von Peptiden der allgemeinen Formel

Arg - Lys - S - Val - Y,

in der S Glutaminsäure oder α-Aminoadipinsäure und Y Tyrosin oder Tryptophan bzw. deren Ester oder Amide, bedeuten, das dadurch gekennzeichnet ist, daß man Tetrapeptide der Formel

Z-Arg($Z'_2$)-Lys(Z')-S-(Bzl)-Val-OH ,

worin Z' für eine Schutzgruppe vom Benzyltyp steht, mit entsprechenden Tyrosin-oder Tryptophanestern bzw. -amiden kondensiert und die Schutzgruppen abhydriert. Die Erfindung betrifft weiterhin Tetrapeptide als Zwischenprodukte dieses Verfahrens.

Croydon Printing Company Ltd.

# Verfahren zur Herstellung von Pentapeptiden mit Wirkung auf das Immunsystem und Zwischenprodukte dieses Verfahrens

Aus dem US-Patent 4 420 424 sind Peptide der allgemeinen Formel

A - B - S - X - Y

in welcher

A Arginin, Lysin, Ornithin oder Homoarginin, jeweils in L- oder D-Konfiguration, ω-Amino-, -Guanidino- oder-Dimethylamino-alkanoyl mit 3 bis 6 C-Atomen und gegebenenfalls einer α-Aminogruppe, in D- oder L-Konfiguration, die ihrerseits Alkanoyl mit 1 bis 6 C-Atomen, Aroyl mit 7 bis 11 C-Atomen, Cycloalkanoyl mit bis zu 2 Alkyl- und 5 bis 7 Cycloalkyl-C-Atomen, Aralkanoyl mit bis zu insgesamt 9 C-Atomen, wobei eine $-CH_2-$Gruppe durch -O- oder -S- ersetzt sein kann, Alkyl- oder Aralkyl-oxycarbonyl mit bis zu 7 C-Atomen oder Succinoyl, Succinamoyl, Glutaroyl, Glutaminyl, Pyroglutamyl, Phthaloyl, Phthalamidyl oder 2-Carboxybenzoyl tragen kann,

B eine basische Aminosäure, vorzugsweise L-Lysin, L-Arginin, L-Homoarginin oder L-Ornithin

S L-Glutaminsäure, D-Glutaminsäure, D-Asparaginsäure, D-α-Aminoadipinsäure

X L-Valin oder L-Isoleucin und

Y eine Aminosäure mit hydrophober Seitenkette in L- oder D-Konfiguration, wie z.B. Tryptophan oder Tyrosin sowie deren Ester, Amid, Alkylamid mit 1 bis 6 C-Atomen oder Aralkylamid mit 7 bis 10 C-Atomen, Alkylamid oder Alkylester mit 1 bis 6 C-Atomen oder Aralkylamid oder Aralkylester mit 7 bis 10 C-Atomen bedeuten, und Verfahren zu deren Herstellung bekannt.

Vor allem Tryptophan, aber auch Tyrosin, neigen bei den Synthesebedingungen bekannter Verfahren zur Bildung von Nebenprodukten.

Es ergab sich somit die Aufgabe, nach nebenproduktarmen Synthesewegen zur Herstellung von Peptiden, die Tryptophan bzw. Tyrosin enthalten, zu suchen.

Diese Aufgabe wurde gelöst durch das erfindungsgemäße Verfahren zur Herstellung von Peptiden der allgemeinen Formel

Arg - Lys - S - Val - Y ,

in der

S Glutaminsäure oder α-Aminoadipinsäure jeweils in der L bzw. D-Konfiguration und

Y Tyrosin oder Tryptophan jeweils in der L- bzw. D-Konfiguration bzw. deren Ester, Amide, Alkylamide, Cycloalkylamide oder Aralkylamide bedeuten, das dadurch gekennzeichnet ist, daß man Tetrapeptide der Formel

$$Z\text{-}Arg(Z'_2)\text{-}Lys(Z')\text{-}S(Bzl)\text{-}Val\text{-}OH ,$$

worin S wie oben definiert ist und Z' für eine Aminoschutzgruppe vom Benzyltyp steht, mit entsprechenden Tyrosin- oder Tryptophanestern bzw. -amiden kondensiert und die Schutzgruppen vom Benzyltyp durch katalytische Hydrierung oder durch katalytische Transferhydrierung beispielsweise mit Ameisensäure abspaltet. Bzl steht für Benzyl oder modifizierte Benzylesterschutzgruppen wie p-Chlor-, p-Brom- oder p-Nitrobenzyl.

Dieses Konzept erlaubt es, Tryptophan bzw. Tyrosin erst zum Schluß in das Peptid einzuführen. Schema 1 zeigt das Syntheseschema für diese Peptide. Wird C-terminal die freie Carboxylgruppe gewünscht, wird für R die Benzyloxygruppe

empfohlen. Diese Schutzgruppen vom Benzyltyp können gemeinsam abgespalten werden.

Das erfindungsgemäße Syntheseprinzip ist jedoch nicht auf die Synthese von Peptiden der Formel Arg-Lys-S-Val-Y, beschränkt, sondern es ist ganz allgemein anwendbar bei der Synthese von Peptiden der eingangs definierten Formel A-B-S-X-Y, in der Y Tyrosin oder Tryptophan bedeutet.

Schema 1

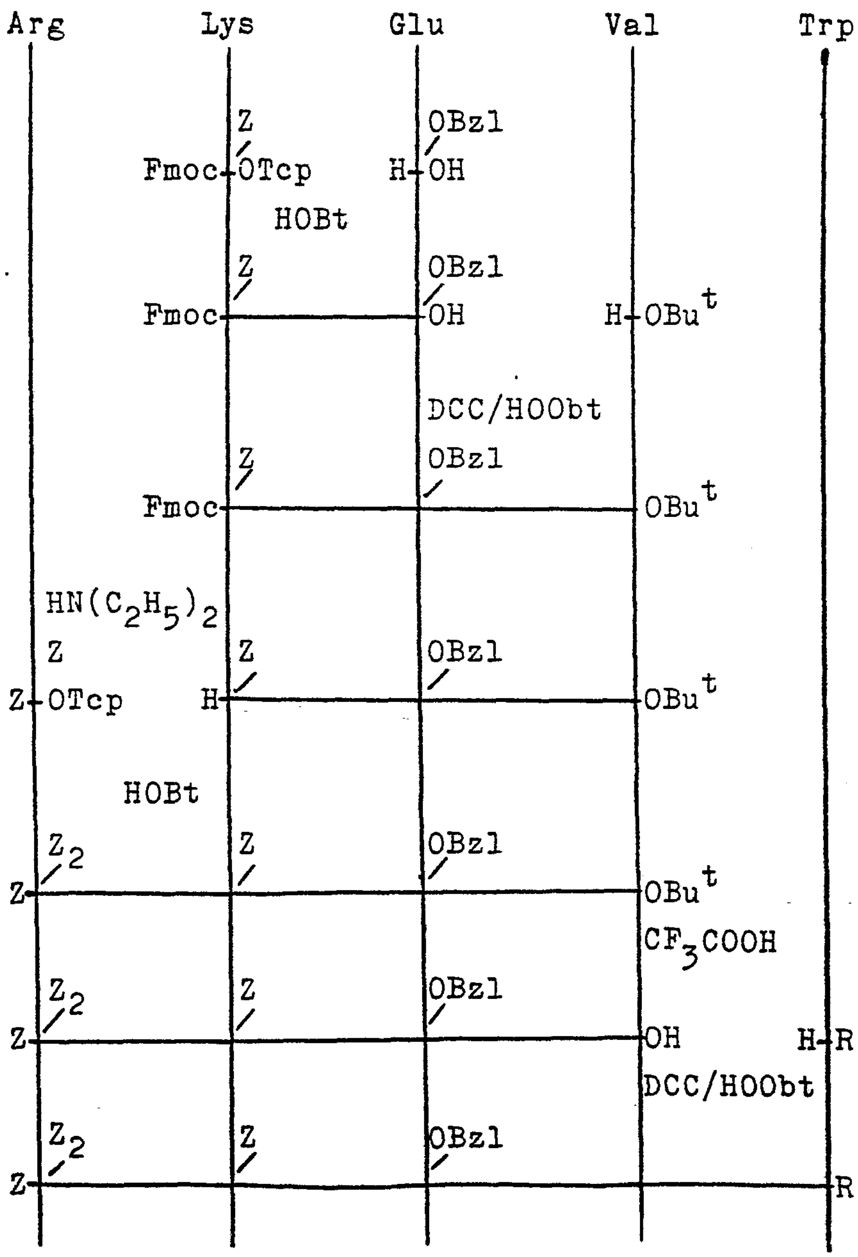

Unter Schutzgruppen vom Benzyltyp (Z') versteht man in diesem Zusammenhang z.B. Benzyloxycarbonyl (Z), $Z(NO_2)$, $Z(Hal)_n$, Moc, insbesondere aber Benzyloxycarbonyl (vgl. hierzu z.B. Hubbuch, Kontakte Merck 3/79, Seite 14 ff.).

C-terminale Estergruppen der Pentapeptide des erfindungsgemäßen Verfahrens sind vorzugsweise ($C_1$-$C_6$)-Alkyl- oder ($C_3$ bis $C_8$)-Cycloalkylestergruppen. Bevorzugt sind auch C-terminale ($C_1$ bis $C_6$)-Alkylamid-, ($C_3$ bis $C_8$)-Cycloalkylamid- und ($C_7$ bis $C_{10}$)-Aralkylamidgruppen (z.B. Benzylamid).

Die Abspaltung der Schutzgruppen Z' erfolgt durch katalytische Hydrierung in DMA, DMF oder Essigsäure (Ber. dtsch. chem. Ges. 65, 1192 (1932) oder durch katalytische Transferhydrierung z.B. mit Ameisensäure (J. Org. Chem. 44, 3442 (1979) oder mit Cyclohexadien (J. Org. Chem. 43, 4194 (1978).

Besonders vorteilhaft erwies sich bei den hier beschriebenen geschützten, schwerlöslichen Peptiden vor allem die katalytische Transferhydrierung in Ameisensäure/Dimethylformamid- oder Dimethylacetamid-Mischungen. So war z.B. Z-Arg($Z_2$)-Lys(Z)-Glu(OBzl)-Val-Trp-OBzl durch katalytische Hydrierung nicht ohne weiteres zu deblockieren. Mit Palladium in Dimethylformamid/Ameisensäure-Lösung wurden dagegen die Schutzgruppen problemlos entfernt.

Zur Kondensation verwendet man Methoden, bei denen die Gefahr der Racemisierung gering ist. Verwendet wurde hier die Dicyclohexylcarbodiimid/3-Hydroxy-4-oxo-3,4-dihydro-1,2,3-benzotriazin (DCC/HOObt)-Methode (Chem. Ber. 103, 2034 (1970)). Aber auch andere racemisierungssenkende Zusätze zum DCC, wie z.B. 1-Hydroxybenzotriazol (Chem. Ber. 103, 788 (1970)), N-Hydroxysuccinimid (Z. Naturforsch. 21b,

426 (1966)) oder N-Hydroxy-5-norbornen-endo-2,3-dicarboximid (Chem. Pharm. Bull. 22, 1857 (1974)) sind möglich.

Nach Abspaltung der Schutzgruppen werden die so gebildeten Peptidformiate beispielsweise durch Behandeln mit schwach basischen Ionen-Austauschern (in Acetat-Form) in die Acetate überführt, die dann wie üblich gereinigt werden können.

Die Erfindung betrifft auch Tetrapeptide der Formel Z'-Arg(Z'$_2$)-Lys(Z')-S(OBzl)-Val-OH, in der S und Z' wie oben definiert sind, sowie Verfahren zu deren Herstellung durch Fragmentkondensation.

Die erfindungsgemäßen Peptide besitzen in vitro in Anwesenheit von Leberhomogenaten gegenüber den natürlichen Thymuspeptiden eine wesentlich verlängerte Lebensdauer. Ihre Wirkung kann z.B. in vitro durch die Beeinflussung von SRBC-Rosetten-bildenen T-Lymphocyten aus Blut von immundefizienten Patienten oder humanem Nabelschnurblut analog J. Exptl- Med. 136 (1972) Seite 207; Anm. N.Y. Acad. Sci. 249 (1975) Seite 308 und Int. Archs.Allergy appl. Immun. 53 (1977) Seite 242, sowie der PHA-induzierten Blastentransformation von humanen und animalischen Lymphocyten analog J. Exptl. Med 131 (1970), Seite 1049, und Cell. Immunol. 16 (1975). Seite 413, nachgewiesen werden. (SRBC = sheep red blood cell; PHA = Phythämagglutinin).

Die erfindungsgemäßen Verbindungen können zur Behandlung von Immundefizienzen, viralen und fungoiden, sowie chronisch bakteriellen Infekten, Autoimmunkrankheiten, sowie zur Therapie von Erkrankungen dienen, die durch Zellen mit immunologisch relevanten Veränderungen der Zellmembranmerkmale (z.B. Tumorzellen) verursacht werden.

In diesem Sinne ist Gegenstand der Erfindung auch die Verwendung der genannten Peptide ganz allgemein zur Beeinflussung der Reifung von T-Lymphozyten.

Die nun folgenden Beispiele dienen zur Erläuterung der Erfindung, ohne daß diese darauf beschränkt wäre.

Beispiel 1:

a) Fmoc-Lys(Z)-OH

Zu einer Suspension von 28 g (100 mmol) H-Lys(Z)-OH und 16,8 g (200 mmol) $NaHCO_3$ in einer Mischung aus 200 ml Wasser und 200 ml Dioxan gibt man 35,38 g (105 mmol) Fmoc-ONSu. Man rührt bei Raumtemperatur ca. 24 Stunden. Man saugt von Ungelöstem ab und engt das Filtrat ein. Der Rückstand wird zwischen 300 ml 1N HCl und Essigsäureethylester verteilt. Die Essigesterphase wird mit Wasser ausgeschüttelt, über $Na_2SO_4$ getrocknet und eingeengt. Das resultierende Öl wird in 200 ml Ether gelöst. Durch Zusatz von 200 ml Petroläther kristallisiert das Produkt. Der Niederschlag wird abgesaugt und nochmals mit Ether verrieben.

Ausbeute: 34,05 g (68 %), Schmp. 110-112°C, $[\alpha]_D^{22} = + 6,5°$ (c = 1, in Tetrahydrofuran).

b) Fmoc-Lys(Z)-OTcp

Zu einer Lösung von 12,55 g (25 mmol) Fmoc-Lys(Z)-OH und 4,92 g (25 mmol) 2,4,5-Trichlorphenol in 100 ml Tetrahydrofuran gibt man bei 0°C 5,76 g (28 mmol) DCC. Man läßt eine Stunde bei 0°C rühren und über Nacht bei Raumtemperatur stehen. Der Niederschlag wird abgesaugt und das Filtrat eingeengt. Das resultierende Öl wird zwei mal mit Petroläther digeriert. Die inzwischen halbfeste Substanz wird mit Ether verrieben, abgesaugt und getrocknet.

Ausbeute 12,36 g (72 %), Schmp. 107°-108°C, $[\alpha]_D^{22}$ = -15,4° (c=1, in Essigester).

c) Fmoc-Lys(Z)-Glu(OBzl)-OH

Zu einer Suspension von 6,6 g (26 mmol) H-Glu(OBzl)-OH und 3,5 g HOBt in 50 ml Dimethylformamid gibt man unter Rühren bei Raumtemperatur 17,7 g (26 mmol) Fmoc-Lys(Z)-OTcp. Man rührt 4 Stunden bei Raumtemperatur und läßt über Nacht stehen. Die Lösung wird eingeengt und der Rückstand mit 150 ml Ethanol in der Wärme gelöst. Man kühlt ab und saugt den Niederschlag ab. Zur weiteren Reinigung wird der Niederschlag mit Wasser verrührt. Anschließend wird abgesaugt und im Vakuum über $P_2O_5$ getrocknet.

Ausbeute: 15 g (79,9 %), Schmp. 156°C, $[\alpha]_D$ = -9,6° (c=1, in Methanol).

d) Fmoc-Lys(Z)-Glu(OBzl)-Val-OBu$^t$

Zu einer Lösung von 14,4 g (20 mmol) Fmoc-Lys(Z)-Glu(OBzl)-OH, 3,26 g HOObt und 4,2 g H-Val-OBu$^t$·HCl in 50 ml Dimethylformamid gibt man bei 0°C 2,6 ml N-Ethylmorpholin und 4,4 g DCC. Man läßt zwei Stunden bei 0°C rühren und läßt über Nacht bei Raumtemperatur stehen. Der Niederschlag wird abgesaugt und das Filtrat mit 150 ml Wasser und 10 ml ges. $NaHCO_3$-Lösung versetzt. Dieser zweite Niederschlag wird abgesaugt, mit Wasser gewaschen und getrocknet. Ausbeute 18 g.

Zur weiteren Reinigung wird die Substanz an 150 g Kieselgel gereinigt. Man eluiert zunächst mit Methylenchlorid und gibt dann wenig Methanol (1-3 %) zum Elutionsmittel zu. Ausbeute: 12,55 g (71.5 %), Schmp. 181-182°C, $[\alpha]_D$ = -35° (c=1, in Methanol).

e) $Z-Arg(Z_2)-Lys(Z)-Glu(OBzl)-Val-OBu^t$

Zu einer Lösung von 12,28 g (14 mmol) $Fmoc-Lys(Z)-Glu(OBzl)-Val-OBu^t$ in 140 ml Dimethylformamid gibt man 14,5 ml (140 mmol) Diethylamin zu. Man rührt 5 Minuten bei Raumtemperatur und engt ein. Der Rückstand wird an Kieselgel chromatographiert. Zunächst eluiert man lipophile Nebenprodukte mit Methylenchlorid. Mit einer Mischung aus Methylenchlorid/Methanol/Wasser wie 9:1:0,1 wird die Substanz eluiert und die entsprechenden Fraktionen eingeengt.
Ausbeute: 9,2 g

Der Rückstand wird zusammen mit 2 g HOBt in 40 ml Dimethylformamid gelöst. In diese Lösung gibt man unter Rühren 12,5 g $Z-Arg(Z_2)-OTcp$. Nach einigen Stunden wird der Ansatz fest. Mit 120 ml Wasser und 14 ml gesättigter $NaHCO_3$-Lösung wird der Brei verrührt. Der Niederschlag wird abgesaugt, mit Wasser gewaschen und über $P_2O_5$ getrocknet. Zur Reinigung wird die Substanz mit Methanol aufgekocht und abgesaugt.

Ausbeute 15,5 g (91 %), Schmp. 184-185°C, $[\alpha]_D = -9,2°$ (c=1, in Dimethylformamid).

f) $Z-Arg(Z_2)-Lys(Z)-Glu(OBzl)-Val-OH$

15 g (12,36 mmol) $Z-Arg(Z_2)-Lys(Z)-Glu(OBzl)-Val-OBu^t$ werden in 150 ml 90%ige (wäßrige) Trifluoressigsäure gelöst. Nach einer Stunde engt man ein. Der Rückstand wird mit Ether verrieben, abgesaugt und getrocknet.
Ausbeute 15,5 g.

Zur Reinigung wird die Substanz mit 200 ml Essigester aufgekocht, abgesaugt und getrocknet.

Ausbeute 11,9 g (83,2 %), Schmp. 177-178°C, $[\alpha]_D$ = -10,2° (c=1, Ameisensäure).

g) Z-Arg($Z_2$)-Lys(Z)-Glu(OBzl)-Val-Trp-OBzl

Zu einer Lösung von 15 g (12,96 mmol) Z-Arg($Z_2$)-Lys(Z)-Glu(OBzl)-Val-OH, 2,11 g HOObt und 6,05 g H-Trp-OBzl·Tos-OH in 130 ml Dimethylformamid gibt man bei 0°C 1,69 ml N-Ethylmorpholin und 2,85 g DCC. Man rührt zwei Stunden bei 0°C und anschließend vier Stunden bei Raumtemperatur. Man läßt über Nacht bei Raumtemperatur stehen und saugt den Niederschlag ab. Das Filtrat wird mit 650 ml Wasser und 13 ml ges. $NaHCO_3$-Lösung versetzt. Der Niederschlag wird abgesaugt, mit Wasser gewaschen und getrocknet.
Ausbeute 19,4 g.

Zur Reinigung wird die Substanz mit 500 ml Essigester aufgekocht, abgesaugt und getrocknet.

Ausbeute 15,1 g (81,3 %), Schmp. 203-204°, $[\alpha]_D$ = -18,1° (c=1, Ameisensäure).

h) H-Arg-Lys-Glu-Val-Trp-OH· $CH_3COOH$

Zu einer Lösung von 10 g (ca. 7 mmol) Z-Arg($Z_2$)-Lys(Z)-Glu(OBzl)-Val-Trp-OBzl in 50 ml Ameisensäure gibt man 50 ml Dimethylformamid. Man überlagert mit $N_2$ und gibt Pd/Kohle-Katalysator zu. Falls die Reaktion nicht richtig anspringt, gibt man nach einiger Zeit nochmals Katalysator zu. Nachdem alle Schutzgruppen abgespalten sind (2 bis 3 Stunden), wird der Katalysator abgesaugt und das Filtrat eingeengt. Der Rückstand wird in Wasser gelöst und mit Ionenaustauscher (IRA 93, Acetat-Form) verrührt. Der Ionenaustauscher wird abgesaugt und zum Schluß mit wenig verdünnter Essigsäure gewaschen bis das Eluat peptidfrei ist. Das Eluat wird eingeengt oder gefriergetrocknet und der Rückstand säulenchromatographisch gereinigt.

Ausbeute ca. 3,5 g, $[\alpha]_D$ = -31,8° (c=1, in Wasser).

**Beispiel 2:**

**a) Z-Arg($Z_2$)-Lys(Z)-Glu(OBzl)-Val-Trp-isobutylamid**

Zu einer Lösung von 1,16 g (1 mmol) Z-Arg($Z_2$)-Lys(Z)-Glu(OBzl)-Val-OH, 0,296 g (1 mmol) H-Trp-isobutylamid-hydrochlorid und 0,163 g (1 mmol) HOObt gibt man bei 0°C 0,13 ml N-Ethylmorpholin und 0,22 g DCC. Man rührt zwei Stunden bei 0°C und läßt anschließend bei Raumtemperatur über Nacht stehen. Der Ansatz erstarrt gallertig. Mit 50 ml Wasser und 1 ml ges. $NaHCO_3$ wird der Ansatz verrührt und abgesaugt. Mit Wasser wird gewaschen und über $P_2O_5$ getrocknet.
Ausbeute: 1,55 g.

Danach wird mit Essigester aufgekocht, abgekühlt und abgesaugt.
Ausbeute 1,42 g (Die Substanz enthält noch Dicyclohexylharnstoff), Schmp. 210-212°C, $[\alpha]_D$ = -10,4° (c=1, in Dimethylformamid).

**b) Arg-Lys-Glu-Val-Trp-isobutylamid-diacetat**

1,4 g der unter Beispiel 2a gewonnenen Substanz (ca. 0,87 mmol Z-Arg($Z_2$)-Lys(Z)-Glu(OBzl)-Val-Trp-isobutylamid) werden in 100 ml Dimethylacetat in der Wärme gelöst. Dazu gibt man 3,34 ml Essigsäure, 10 ml Wasser und Pd/Kohle-Katalysator. Wasserstoff wird durch die Lösung geleitet. Nach Beendigung der Hydrierung (DC-Kontrolle) wird der Katalysator abgesaugt und das Filtrat eingeengt. Der Rückstand wird säulenchromatographisch gereinigt.

Ausbeute 359 mg (Peptidbasengehalt lt. Aminosäureanalyse: ca. 70 %) (46 %). $[\alpha]_D$ = -34,7° (c=1, Wasser).

Beispiel 3:

a) Z-Arg($Z_2$)-Lys(Z)-Glu(OBzl)-Val-Trp-cyclohexylester

1,16 g (1 mmol) Z-Arg($Z_2$)-Lys(Z)-Glu(OBzl)-Val-OH und 0,323 g (1 mmol) H-Trp-cyclohexylester-hydrochlorid werden analog Beispiel 2a umgesetzt. Der ausgefallene Dicycloheylharnstoff wird abgesaugt und das Filtrat mit 50 ml Wasser und 1 ml einer wäßrigen ges. $NaHCO_3$-Lösung versetzt. Weitere Aufarbeitung analog Beispiel 2a.

Ausbeute 1,23 g (86 %), Schmp. 205-206°C, $[\alpha]_D$ = -5,6° (c=1, in Dimethylformamid).

b) Arg-Lys-Glu-Val-Trp-cyclohexylester-diacetat

1,2 g (0,84 mmol) Z-Arg($Z_2$)-Lys(Z)-Glu(OBzl)-Val-Trp-cyclohexylester werden analog Beispiel 2b katalytisch hydriert und gereinigt.

Ausbeute 505 mg (Peptidgehalt lt. Aminosäureanalyse: ca. 70 %) (44 %), $[\alpha]_D$ = -36,3° (c=1, in Wasser).

Beispiel 4:

a) Fmoc-Lys(Z)-D-Aad(OBzl)-OH

Zu einer Suspension von 15,7 g (62,5 mmol) H-Aad(OBzl)-OH in 125 ml Dimethylformamid gibt man 8,5 g HOBt und 42,7 g (62,5 mmol) Fmoc-Lys(Z)-OTcp. Man läßt 8 Stunden bei Raumtemperatur und über zwei Tage bei 5°C rühren. Anschließend ist der Ansatz erstarrt. Man verreibt mit 800 ml Diethylether, saugt ab und wäscht mit Ether nach.
Ausbeute 35,9 g (78 %), Schmp. 138-139°C.

b) Fmoc-Lys(Z)-D-Aad(OBzl)-Val-$OBu^t$

Zu einer Suspension von 34,95 g (47,5 mmol) Fmoc-Lys(Z)-D-Aad(OBzl)-OH, 9,98 g (47,5 mmol) H-Val-$OBu^t$, 9,98 g

(47,5 mmol) H-Val-$OBu^t$·HCl und 7,75 g HOObt in 200 ml Dimethylformamid gibt man bei 0°C 6,2 ml N-Ethylmorpholin und 10,45 g DCC in 50 ml Dimethylformamid. Man arbeitet analog Beispiel 1d auf. Zur Reinigung wird die noch feuchte Substanz in 300 ml heißem Essigester gelöst. Man trocknet die Essigesterlösung in der Wärme mit $Na_2SO_4$ und filtriert heiß von $Na_2SO_4$ ab. Mit 900 ml Petrolether wird aus der Essigesterlösung eine Substanz ausgefällt. Der Niederschlag wird abgesaugt und mit 300 ml Isopropanol heiß gelöst. Man läßt über Nacht bei 4°C stehen und saugt den Niederschlag ab.

Ausbeute 31 g (87,1 %), Schmp. 120-122°C.

c) Z-Arg($Z_2$)-Lys(Z)-D-Aad(OBzl)-Val-$OBu^t$

30,34 g (40,5 mmol) Fmoc-Lys(Z)-D-Aad(OBzl)-Val-$OBu^t$ werden analog Beispiel 1e mit 162 ml (405 mmol) Diethylamin in 405 ml Dimethylformamid behandelt.

Ausbeute 18,5 g (68,3 %).

Dieser Rückstand wird analog Beispiel 1e mit 21 g (27,6 mmol) Z-Arg($Z_2$)-OTcp und 3,7 g HOBt umgesetzt. Der Ansatz erstarrt nach zwei Stunden gallertig. Er wird mit 200 ml Wasser und 28 ml ges. $NaHCO_3$-Lösung verrieben, abgesaugt und mit Wasser gewaschen. Anschließend wird mit 300 ml Methanol aufgekocht und abgesaugt.

Ausbeute 23,2 g (68,3 %), Schmp. 171-172°C, $[\alpha]_D$ = +0,9° (c=1, in Dimethylformamid).

d) Z-Arg($Z_2$)-Lys(Z)-D-Aad(OBzl)-Val-OH

22,7 g (18.5 mmol) Z-Arg($Z_2$)-Lys(Z)-D-Aad(OBzl)-Val-OH werden in 227 ml 90%iger Trifluoressigsäure gelöst. Man läßt eine Stunde bei Raumtemperatur stehen und engt ein. Der Rückstand wird mit Ether verrieben, abgesaugt und getrocknet.

Ausbeute 19,1 g.

Zur Reinigung wird die Substanz mit 200 ml Methanol aufgekocht, abgesaugt und getrocknet.
Ausbeute 16,1 g (74,3 %), Schmp. 167-168°C, $[\alpha]_D$ = -3,8° (c=1, Ameisensäure).

e) Z-Arg($Z_2$)-Lys(Z)-D-Aad(OBzl)-Val-Trp-isobutylamid

1,17 g (1 mmol) Z-Arg($Z_2$)-Lys(Z)-D-Aad(OBzl)-Val-OH werden analog Beispiel 2a mit 0,296 g H-Trp-isobutylamid-hydrochlorid umgesetzt. Der Dicyclohexylharnstoff wird abgesaugt und das Filtrat mit 50 ml Wasser und 1 ml ges. $NaHCO_3$-Lösung versetzt. Der Niederschlag wird abgesaugt, mit Wasser gewaschen und getrocknet. Zur Reinigung wird mit 50 ml Methanol aufgekocht, abgesaugt und getrocknet.
Ausbeute 1,21 g (85,6 %), Schmp. 203-206°C, $[\alpha]_D$ = -11,2° (c=1, Dimethylformamid).

f) Arg-Lys-D-Aad-Val-Trp-isobutylamid-diacetat

1 g Z-Arg($Z_2$)-Lys(Z)D-Aad(OBzl)-Val-Trp-isobutylamid werden in 5 ml Ameisensäure und 5 ml Dimethylformamid gelöst und analog Beispiel 1h umgesetzt und gereinigt.
Ausbeute 213 mg (Gehalt an Peptidbase lt. Aminosäureanalyse 78 %), $[\alpha]_D$ = -5° (c=1, in Wasser).

Beispiel 5:
a) Z-Arg($Z_2$)-Lys(Z)-D-Aad(OBzl)-Val-Trp-cyclohexylester

1,17 g (1 mmol) Z-Arg($Z_2$)-Lys(Z)-D-Aad(OBzl)-Val-OH werden analog Beispiel 3a umgesetzt. Zur Reinigung wird die Substanz mit 50 ml Methanol aufgekocht, abgesaugt und getrocknet.
Ausbeute 1,2 g (83,3 %), $[\alpha]_D$ = -1,3° (c=1, Dimethylformamid).

b) Arg-Lys-D-Aad-Val-Trp-cyclohexylester-diacetat

1,0 g Z-Arg($Z_2$)-Lys(Z)-D-Aad(OBzl)-Val-Trp-cyclohexylester werden analog Beispiel 1h umgesetzt und gereinigt. Ausbeute 343 mg (Gehalt an Peptidbase lt. Aminosäureanalyse: ca. 75 %), $[\alpha]_D$ = -13,4° (c=1, Wasser).

Beispiel 6:

a) Z-Arg($Z_2$)-Lys(Z)-D-Aad(OBzl)-Val-Tyr-OBzl

11,72 g (10 mmol) Z-Arg($Z_2$)-Lys(Z)-D-Aad(OBzl)-Val-OH werden analog Beispiel 1g mit 4,44 g (10 mmol) H-Tyr-OBzl-tosylat umgesetzt. Zur Reinigung wird die Substanz mit 500 ml Methanol aufgekocht. Ausbeute 12,6 g (88,4 %), $[\alpha]_D$ = -0,4° (c=1, Dimethylformamid).

b) Arg-Lys-D-Aad-Val-Tyr-acetat

12 g Z-Arg($Z_2$)-Lys(Z)-D-Aad(OBzl)-Val-Tyr-OBzl werden analog Beispiel 1h umgesetzt und gereinigt. Ausbeute 4,5 g, (Gehalt an Peptidbase lt. Aminosäureanalyse: ca. 83,9 %), $[\alpha]_D$ = +31,7° (c=1, Wasser).

Beispiel 7:

a) Z-Arg($Z_2$)-Lys(Z)-D-Aad(OBzl)Val-Trp-OBzl

1,17 g (1 mmol) Z-Arg($Z_2$)-Lys(Z)-D-Aad(OBzl)-Val-OH werden analog Beispiel 1g mit 0,466 g (1 mmol) H-Trp-OBzl-tosylat umgesetzt. Zur Reinigung wird die Substanz mit 50 ml Methanol aufgekocht, abgesaugt und getrocknet. Ausbeute 1,22 g (84,2 %), $[\alpha]_D$ = +1,8° (c=1, Dimethylformamid).

b) Arg-Lys-D-Aad-Val-Trp-acetat

1 g Z-Arg($Z_2$)-Lys(Z)-D-Aad(OBzl)-Val-Trp-OBzl werden analog Beispiel 1h umgesetzt und gereinigt.
Ausbeute 291 mg (Gehalt an Peptidbase lt. Aminosäureanalyse: 84 %), $[\alpha]_D$ = +3,30 (c=1, Wasser).

Abkürzungen:

| | |
|---|---|
| DCC | Dicyclohexylcarbodiimid |
| HOObt | 3-Hydroxy-4-oxo-3,4-dihydro-1,2,3-benzotriazin |
| HOBt | 1-Hydroxybenzotriazol |
| $OBu^t$ | tert.-Butylester |
| OBzl | Benzylester |
| Fmoc | 9-Fluorenylmethyloxycarbonyl |
| Z | Benzyloxycarbonyl |

**Patentansprüche:**

1. Verfahren zur Herstellung von Peptiden der allgemeinen Formel

Arg - Lys - S - Val - Y ,

in der
S Glutaminsäure oder α-Aminoadipinsäure jeweils in der L bzw. D-Konfiguration und
Y Tyrosin oder Tryptophan jeweils in der L- bzw. D-Konfiguration bzw. deren Ester, Amide, Alkylamide, Cycloalkylamide oder Aralkylamide bedeuten, dadurch gekennzeichnet, daß man Tetrapeptide der Formel

Z-Arg(Z'$_2$)-Lys(Z')-S-(Bzl)-Val-OH ,

worin S wie oben definiert ist und Z' für eine Aminoschutzgruppe vom Benzyltyp steht, mit entsprechenden Tyrosin- oder Tryptophanestern bzw. -amiden kondensiert und die Schutzgruppen vom Benzyltyp durch katalytische Hydrierung oder durch katalytische Transferhydrierung abspaltet.

2. Verfahren nach Anspruch 1, dadurch gekennzeichnet, daß die Abspaltung der Benzylschutzgruppe durch katalytische Transferhydrierung mit Ameisensäure/Dimethylformamid- oder Dimethylacetamid-Mischungen erfolgt.

3. Tetrapeptid Z'-Arg(Z'$_2$)-Lys(Z')-S(OBzl)-Val-OH, in dem S Glutaminsäure oder Aminoadipinsäure in L- bzw. D-Konfiguration bedeutet und Z' für eine Aminoschutzgruppe vom Benzyltyp steht.

4. Verfahren zur Herstellung eines Tetrapeptids gemäß Anspruch 3, dadurch gekennzeichnet, daß dieses durch Fragmentkondensation aufgebaut wird.

5. Peptid der Formel

Arg - Lys - S - Val -Y,

in der S = Glu und Y = Trp oder S = D-Aad und Y = Tyr bedeuten, sowie deren physiologisch verträglichen Salze.

6. Peptid H-Arg-Lys-Glu-Val-Trp-OH, sowie dessen physiologisch verträglichen Salze.

7. Peptid H-Arg-Lys-D-Aad-Val-Tyr-OH, sowie dessen physiologisch verträglichen Salze.

8. Peptid gemäß Anspruch 5, 6 oder 7 zur Anwendung als Heilmittel.

9. Verwendung eines Peptids gemäß Anspruch 5, 6 oder 7 zur Beeinflussung der Reifung von T-Lymphozyten.

10. Pharmazeutisches Mittel enthaltend ein Peptid gemäß einem der Anspruch 5 - 8.

Patentansprüche Österreich

1. Verfahren zur Herstellung von Peptiden der allgemeinen Formel

Arg - Lys - S - Val - Y ,

in der
S Glutaminsäure oder α-Aminoadipinsäure jeweils in der L bzw. D-Konfiguration und
Y Tyrosin oder Tryptophan jeweils in der L- bzw. D-Konfiguration bzw. deren Ester, Amid, Alkylamid, Cycloalkylamid oder Aralkylamid bedeuten, dadurch gekennzeichnet, daß man Tetrapeptide der Formel

Z-Arg(Z'$_2$)-Lys(Z')-S-(Bzl)-Val-OH ,

worin S wie oben definiert ist und Z' für eine Aminoschutzgruppe vom Benzyltyp steht, mit entsprechenden Tyrosin- oder Tryptophanestern bzw. -amiden kondensiert und die Schutzgruppen vom Benzyltyp durch katalytische Hydrierung oder durch katalytische Transferhydrierung abspaltet.

2. Verfahren nach Anspruch 1, dadurch gekennzeichnet, daß die Abspaltung der Benzylschutzgruppe durch katalytische Transferhydrierung mit Ameisensäure/Dimethylformamid- oder Dimethylacetamid-Mischungen erfolgt.

3. Verfahren zur Herstellung eines Tetrapeptids Z'-Arg(Z'$_2$)-Lys(Z')-S(Obzl)-Val-OH, in dem S Glutaminsäure oder Aminoadipinsäure in L- bzw. D-Konfiguration bedeutet und Z' für eine Aminoschutzgruppe vom Benzyltyp steht, dadurch gekennzeichnet, daß dieses durch Fragmentkondensation aufgebaut wird.

4. Verfahren gemäß Anspruch 1 oder 2, dadurch gekennzeichnet, daß man ein Peptid der Formel

Arg - Lys - S - Val -Y,

in der S = Glu und Y = Trp oder S = D-Aad und Y = Tyr bedeuten, herstellt und dieses gegebenenfalls in ein physiologisch verträgliches Salz überführt.

5. Verfahren gemäß Anspruch 4, dadurch gekennzeichnet, daß man das Peptid H-Arg-Lys-Glu-Val-Trp-OH oder dessen physiologisch verträgliches Salz herstellt.

6. Verfahren gemäß Anspruch 4, dadurch gekennzeichnet, daß man das Peptid H-Arg-Lys-D-Aad-Val-Tyr-OH oder dessen physiologisch verträgliches Salz herstellt.

7. Verwendung eines Peptids hergestellt gemäß einem der Ansprüche 4 - 6, zur Beeinflussung der Reifung von T-Lymphozyten.

8. Verfahren zur Herstellung eines pharmazeutischen Mittels enthaltend ein Peptid hergestellt gemäß einem der Ansprüche 4 - 6, dadurch gekennzeichnet, daß man dieses in eine geeignete Darreichungsform bringt.